# EUROPÄISCHE PATENTSCHRIFT

(11) **EP 2 493 915 B1**
(45) Veröffentlichungstag und Bekanntmachung des Hinweises auf die Patenterteilung: **16.08.2017**
(21) Anmeldenummer: 10773290.1
(22) Anmeldetag: 25.10.2010
(51) Int. Cl.: C07K 14/47

(54) **STABILISIERUNG VON BIOSENSOREN FÜR IN VIVO ANWENDUNGEN**
Stabilisation of biosensors for in vivo applications
Stabilisation de biocapteurs pour applications in vivo

(30) Priorität: 26.10.2009 EP 09174054
(43) Veröffentlichungstag der Anmeldung: 05.09.2012
(73) Patentinhaber: EyeSense AG, 4051 Basel (CH)
(72) Erfinder: HERBRECHTSMEIER, Peter, 61462 Königstein (DE); MÜLLER, Achim, 63762 Grossostheim (DE); KNUTH, Monika, 63773 Goldbach (DE); NIKOLAUS, Katharina, 63739 Aschaffenburg (DE)
(74) Vertreter: Herzog, Fiesser & Partner Patentanwälte PartG mbB
(86) Internationale Anmeldenummer: PCT/EP2010/066036
(87) Internationale Veröffentlichungsnummer: WO 2011/054690

(56) Entgegenhaltungen:
- EP-A2- 0 602 686
- WO-A1-97/24136
- GB-A- 1 488 988
- US-A- 5 962 414
- JONES D B AND JOHNS C O: "SOME PROTEINS FROM THE JACK BEAN, CANAVALIA ENSIFORMIS", JOURNAL OF BIOLOGICAL CHEMISTRY, Bd. 28, 1916, Seiten 67-75, XP002560827,
- BALLERSTADT RALPH ET AL: "Concanavalin A for in vivo glucose sensing: A biotoxicity review", BIOSENSORS & BIOELECTRONICS, Bd. 22, Nr. 2, August 2006 (2006-08), Seiten 275-284, XP002560828, ISSN: 0956-5663
- RUSSEL ET AL: "A Fluorescence-Based Glucose Biosensor Using Concanavalin A and Dextran Encapsulated in a Poly(ethylene glycol) Hydrogel", 19990801, Bd. 71, Nr. 15, 1. August 1999 (1999-08-01), Seiten 3126-3132, XP002322664,
- CARLINI C R ET AL: "Isolation and characterization of a toxic protein from Canavalia ensiformis (jack bean) seeds, distinct from concanavalin A", TOXICON, ELMSFORD, NY, US, Bd. 19, Nr. 5, 1. Januar 1981 (1981-01-01) , Seiten 667-675, XP023762703, ISSN: 0041-0101 [gefunden am 1981-01-01]
- KAMRA A ET AL: "Reaction of concanavalin A with dimethyl adipimidate: purification and characterization of a crosslinked concanavalin a derivative with enhanced thermal stability", BIOCHIMICA ET BIOPHYSICA ACTA - GENERAL SUBJECTS, ELSEVIER SCIENCE PUBLISHERS, NL, Bd. 966, Nr. 2, 11. August 1988 (1988-08-11), Seiten 181-187, XP023499822, ISSN: 0304-4165
- CHEBOTAREVA N A ET AL: "Biochemical effects of molecular crowding", BIOCHEMISTRY (MOSCOW), Bd. 69, Nr. 11, November 2004 (2004-11), Seiten 1239-1251, XP002560829, ISSN: 0006-2979 in der Anmeldung erwähnt
- MATTO MAHREEN ET AL: "Entrapment of porous and stable concanavalin A-peroxiaase complex into hybrid calcium alginate-pectin gel", JOURNAL OF CHEMICAL TECHNOLOGY AND BIOTECHNOLOGY, vol. 81, no. 7, July 2006 (2006-07), pages 1316-1323, ISSN: 0268-2575
- KAMRA A ET AL: "Reaction of concanavalin A with dimethyl adipimidate: purification and characterization of a crosslinked concanavalin a derivative with enhanced thermal stability", BIOCHIMICA ET BIOPHYSICA ACTA - GENERAL SUBJECTS, ELSEVIER SCIENCE PUBLISHERS, NL, vol. 966, no. 2, 11 August 1988 (1988-08-11), pages 181-187, XP023499822, ISSN: 0304-4165, DOI: 10.1016/0304-4165(88)90110-9 [retrieved on 1988-08-11]
- Stephanie Campbell Smith ET AL: "Biochemical Characterization of Canavalin, the Major Storage Protein of Jack Bean 1", Plant Physiology, 1 October 1982 (1982-10-01), pages 1199-1209, XP55275937, Retrieved from the Internet: URL:http://www.plantphysiol.org/content/70 /4/1199.full.pdf

## Beschreibung

Die vorliegende Erfindung betrifft die Verwendung von Zubereitungen für die Stabilisierung isolierter Proteine. Insbesondere wird die Verwendung solcher Zubereitung zur Stabilisierung von Rezeptoren in biochemischen Sensoren offenbart. Die Erfindung betrifft zudem biochemische Sensoren mit solchen Zubereitungen . Ein biochemischer Sensor zur Bestimmung der Glukosekonzentration, welcher Concanavalin A zusammen mit Dextran in Hydrogelpartikeln enthält, wurde von Russell beschrieben (Russell et al., 1999, Anal. Chem., 71: 3126-3132). Vielfach ist es für die technische Anwendung von Proteinen wünschenswert, diese von störenden Verunreinigungen zu befreien. Ein wichtiges Einsatzgebiet aufgereinigter Proteine sind biochemische Sensoren. Proteine, die als Rezeptoren biochemischer Sensoren verwendet werden, müssen eine hohe Affinität zum Analyten aufweisen. Um die Bindung des Analyten an den Rezeptor nachzuweisen wird der Rezeptor häufig modifiziert, um die Wechselwirkung von Analyt und Rezeptor messbar zu machen. Eine gezielte Modifizierung wird durch Verunreinigungen erschwert und vielfach unmöglich gemacht. Deswegen werden Proteine aus natürlichen Zellen und Geweben extrahiert und aufgereinigt.

Die Aufreinigung von Proteinen hat allerdings den Nachteil, dass die Stabilität des isolierten Proteins häufig geringer ist als seine Stabilität in der natürlichen Umgebung. Die Zugabe von Chaperonen, Aminosäuren, Proteinen (z.B. BSA), Polysacchariden oder Polysaccharidderivaten erhöht vielfach die Stabilität isolierter Proteine. Häufig werden nieder- oder hochmolekulare Zusätze verwendet, um das freie Volumen in der Lösung zu verringern ("molecular crowding"). Dabei können Proteine, Polysaccharide oder synthetische Polymere benutzt werden. Aus dem Stand der Technik sind insbesondere Polyethylenglycol, Ficoll, Dextran, Ribonuklease und bovines Serumalbumin bekannt. Der Effekt dieser Zugaben kann durch den Effekt des "ausgeschlossenen Volumens" erklärt werden (Chebotareva et al., 2004, Biochemical Effects of Molecular Crowding, Biochemistry (Moscow), 69: 1522-1536) Weiterhin kann Kreuzvernetzung die Stabilität eines Proteins, z. B. Concanavalin A, erhöhen (Kamra et al., 1988, Biochimica et Biophysica Acta, 966: 181-187). Aufgabe der Erfindung ist also die Bereitstellung von Zubereitungen, die eine verbesserte Stabilisierung isolierter Proteine ermöglichen. Die Aufgabe wird durch die Ausführungsformen gelöst, die in den Patentansprüchen beschrieben werden.

Die Erfindung betrifft somit die Verwendung einer Zelllysatfraktion zur Stabilisierung eines isolierten Proteins, wobei die Zelllysatfraktion durch ein Verfahren gewonnen wird, das die Schritte umfasst:
a) Gewinnung des Zelllysats aus Zellen; und
b) Abtrennung des isolierten Proteins aus dem Zelllysat, wodurch die Zellysatfraktion zur Stabilisierung des isolierten Proteins erhalten wird.

Bevorzugt werden das isolierte Protein und die Zelllysatfraktion aus *Canavalia ensiformis* gewonnen. Besonders bevorzugt enthält die Zelllysatfraktion Präcanavalin. Bevorzugt wird die Zelllysatfraktion nach der Abtrennung des isolierten Proteins weiter aufgearbeitet. Bevorzugt ist das das isolierte Protein ein Rezeptor, ganz besonders bevorzugt Concanavalin A. Bevorzugt ist das isolierte Protein Bestandteil eines biochemischen Sensors. Besonders bevorzugt umfasst der biochemische Sensor zusätzlich Dextran und Concanavalin in Hydrogelpartikeln.

Die Zellysatfration, die erfindungsgemäß eingesetzt wird, ist erhältlich durch ein Verfahren zur Bereitstellung einer ein isoliertes Protein stabilisierenden Zelllysatfraktion umfassend die Schritte
a) Gewinnung des Zelllysats aus den Zellen; und
b) Abtrennung des zu isolierenden Proteins aus dem Zelllysat.

Unter "Zellen" werden in dieser Anmeldung alle prokaryotischen oder eukaryotischen Zellen verstanden, die das zu isolierende Protein exprimieren. Dies schließt genetisch transformierte Zellen ein, in welchen das zu isolierende Protein rekombinant exprimiert wird. Die Zellen können als Einzelzellen einer prokaryotischen oder eukaryotischen Zellkultur vorliegen oder in Form einer Gewebeprobe eines tierischen oder pflanzlichen Organismus oder eines Pilzes.

Verfahren zur Gewinnung eines Zelllysats sind dem Fachmann gut bekannt. Die Zellen können im Rahmen des erfindungsgemäßen Verfahrens mit allen bekannten mechanischen oder nicht-mechanischen Aufschlussverfahren aufgeschlossen werden. Bevorzugte mechanische Aufschlussverfahren sind die Homogenisierung mit rotierenden Messern (bei tierischen Zellen), das Potter-Elvehjem-Verfahren, das Zermahlen der Zellen oder des Gewebes, das Zerreiben im Mörser mit Sand, Aluminiumoxid oder Glasperlen, der Zellaufschluss durch Kavitationskräfte bei Ultraschall und das Pressen einer Zellsuspension unter hohem Druck durch ein enges Ventil (z.B. in einer French Press). Der Fachmann weiß, dass bei mechanischen Aufschlussverfahren Wärme entstehen kann, so dass in vielen Fällen eine Temperaturregulierung notwendig ist, um eine Denaturierung der Proteine zu verhindern. Bevorzugte nicht-mechanische Aufschlussverfahren sind das wiederholte Einfrieren und Auftauen der Zellen, Behandlung der Zellen mit hypotonischen Löungen, die Behandlung mit Lysozym bei gram-positiven Bakterien, die Behandlung mit EDTA und anschließende Inkubation mit Lysozym bei gram-negativen Bakterien oder die Behandlung mit Toluen bei Hefen. Selbstverständlich können verschiedene der genannten Verfahren untereinander oder mit weiteren hier nicht genannten Aufschlussverfahren kombiniert werden.

Während des Aufschlusses müssen die Proteine vor schädlichen Einflüssen geschützt werden. Bevorzugt werden Kälte oder spezifische Inhibitoren verwendet um den Abbau der Proteine durch Proteasen zu verhindern. Zum Schutz von Thiolgruppen werden bevorzugt Reduktionsmittel, besonders bevorzugt Dithiothreitol oder Dithioerythol, eingesetzt. Zum Schutz vor Schwermetallionen ist Ethylendiamintetraessigsäure (EDTA) bevorzugt. Diese bindet auch zweiwertige Kationen, die Proteasen aktivieren können. Zur Verhinderung der Aggregation von Proteinen werden bevorzugt nicht-ionische Detergenzien verwendet.

Bevorzugt sind das Aufschlussverfahren und ggf. zu treffende Schutzmaßnahmen nicht nur darauf abgestimmt, dass das gewünschte Protein im Zelllysat in aktiver Form vorliegt. Auch die Bestandteile des Zelllysats, welche die Stabilisierung des Proteins vermitteln, dürfen durch das gewählte Aufschlussverfahren nicht in ihrer Funktion beeinträchtigt werden.

Vor ihrer praktischen Anwendung werden isolierte Proteine in vielen Fällen modifiziert. So werden Rezeptoren für den Einsatz in biochemischen Sensoren häufig markiert. Diese Markierungen erfolgen durch die kovalente Modifikation des Rezeptors beispielsweise mit Fluoreszenzfarbstoffen. Damit das modifizierte isolierte Protein, das stabilisiert werden soll, nicht durch das in der Zelllysatfraktion enthaltene nicht modifizierte Protein verdünnt wird, ist es bevorzugt, das zu isolierende Protein aus dem Zelllysat abzutrennen. Der Begriff "Abtrennung des zu isolierenden Proteins aus dem Zelllysat" bezeichnet die Aufreinigung des gewünschten Proteins. Als Ergebnis liegt wenigstens eine Zelllysatfraktion vor, die das Protein als isoliertes Protein enthält. Weiterhin liegt getrennt davon wenigstens eine Zelllysatfraktion vor, die das vorgenannte Protein nicht oder nur in geringem Anteil enthält. Das isolierte Protein ist von seinem natürlichen Kontext, d.h. von den Molekülen, mit denen es im Zelllysat vorlag, getrennt. Vorzugsweise liegt das isolierte Protein bezogen auf die Moleküle aus seiner natürlichen Umgebung zu wenigstens 50 %, wenigstens 75 %, wenigstens 80 %, wenigstens 90 %, wenigstens 95 % oder, besonders bevorzugt, zu wenigstens 99 % (Gewicht/Gewicht) rein vor. Die Zelllysatfraktion, die das isolierte Protein enthält, kann in beliebiger Menge Moleküle enthalten, die nicht der natürlichen Umgebung des besagten Proteins entstammen, sondern im Zuge der Aufreinigung oder nach der Aufreinigung zugefügt wurden. Ein Beispiel hierfür wären Bestandteile des verwendeten Puffers.

Verfahren zur Abtrennung eines zu isolierenden Proteins aus einem Zelllysat sind dem Fachmann bekannt. Bevorzugte Verfahren sind Fällung und differentielle Solubilisierung, Ultrazentrifugation, chromatographische Verfahren und Elektrophorese.

Die Fällung von Proteinen wird bevorzugt durch die Zugabe von Ammoniumsulfat in steigenden Konzentrationen erreicht. Proteinfraktionen verschiedener Löslichkeit fallen bei diesem Verfahren in Abhängigkeit von der erreichten Ammoniumsulfatkonzentration nacheinander aus und können abgetrennt werden. Gleichfalls bevorzugt ist die Ausfällung des Proteins durch Ansäuerung des Medium mit einem geeigneten Puffer.

Ultrazentrifugation beruht auf dem Prinzip, dass im durch die Zentrifugation erzeugten Schwerefeld Partikel umso schneller sedimentieren, je dichter und kompakter sie sind. Als Folge des Gleichgewichts zwischen Zentrifugalkraft und Auftrieb, das sich während einer ausreichend langen Zentrifugation einstellt, reichern sich die einzelnen Proteine an den Stellen im Gefäß an, an denen sich Auftrieb und Zentrifugalkraft gegenseitig ausgleichen. Die Verwendung von Sucrose-Gradienten kann diesen Prozess erleichtern.

Die chromatografische Isolierung von Proteinen beruht auf dem Prinzip, dass die in einer mobilen Phase, bevorzugt einem Puffer, gelösten Proteine über eine stationäre Phase wandern. Die Phasen sind so gewählt, dass die unterschiedlich starke Interaktion einzelner Proteine aus dem Proteingemisch mit der festen Phase zu unterschiedlich langen Laufzeiten der einzelnen Proteinftaktionen über die stationäre Phase führt. Auf welche Art Proteine und stationäre Phase interagieren, hängt von der Art der Chromatographie ab.

Bevorzugte chromatographische Verfahren zur Isolierung von Proteinen sind Größenausschlusschromatographie (basierend auf der unterschiedlichen Größe der aufzutrennenden Proteine), Affinitätschromatographie (basierend auf der Fähigkeit einzelner Proteine, spezifisch an das Säulenmaterial zu binden), Ionenaustauschchromatographie (basierend auf den unterschiedlichen Isoelektrischen Punkten verschiedener Proteine) und Umkehrphasenchromatographie (basierend auf der unterschiedlichen Hydrophobizität verschiedener Proteine).

Der Begriff "isoliertes Protein" bezeichnet im Rahmen dieser Patentanmeldung beliebige Proteine, an deren Isolierung in funktionalen Zustand Interesse besteht. Im Folgenden wird der Begriff "gewünschtes Protein" synonym verwendet. Isolierte Proteine im Sinne der vorliegenden Anmeldung umfassen bevorzugt Enzyme, Rezeptoren, modulatorische Proteine, Transkriptionsfaktoren, Zytoskelettproteine, Bindeproteine und Membrantransporter, besonders bevorzugt Rezeptoren. Ganz besonders bevorzugt ist das isolierte Protein Concanavalin A. Die isolierten Proteine können im Rahmen des Verfahrens als Reinstoffe oder partiell isoliert vorliegen. Bevorzugt umfassen isolierte Proteine oder Präparationen von isolierten Proteinen weniger als 50 %, weniger als 25 %, weniger als 10 %, weniger als 5 %, weniger als 1 % oder weniger als 0,5 % Protein oder Protein und zelluläre Bestandteile als Verunreinigung.

Unter "Enzym" wird jedes Protein verstanden, das in der Lage ist, eine chemische Reaktion zu katalysieren. Dabei spielt es keine Rolle, ob im Rahmen der chemischen Reaktion andere Moleküle umgewandelt werden oder ob das Enzym autokatalytisch wirkt.

Ein "Rezeptor" ist jedes Protein, das in der Lage ist, ein Molekül, den Liganden, spezifisch zu binden und auf diese Bindung mit einer Konformationsveränderung oder einer Aktivitätsänderung zu reagieren. Auch Proteine, die in ihrem natürlichen Zustand keine Reaktion auf die Bindung eines Liganden zeigen, können chemisch modifiziert werden, so dass die Bindung eines Liganden zu messbaren Veränderungen des Proteins führt. Weiterhin sind durch die Kombination eines fluoreszenzmarkierten Rezeptors mit einem ebenfalls fluoreszenmarkierten Liganden kompetetive Assays möglich. In der Abwesenheit von unmarkiertem Liganden in der Probe bindet nur der fluoreszenzmarkierte Ligand an den Rezeptor. Die räumliche Nähe der beiden Fluoreszenzfarbstoffe beeinflusst ihre Lichtemission. Ist nun in einer Probe unmarkierter Ligand vorhanden, so verdrängt der unmarkierte Ligand den fluoreszenzmarkierten Liganden vom Rezeptor und verändert so die Lichtemission des Systems. Die Veränderung der Lichtemission des Systems hängt somit von der Menge an nicht markiertem Liganden ab, der mit der Probe in das System eingebracht wird und markierte Liganden von den Rezeptoren verdrängt. Spezifische Bindung bedeutet, dass der Ligand mit signifikant höherer Affinität gebunden wird als andere Substanzen. Vorzugsweise wird der Ligand mit wenigstens 10-fach, 100-fach, 1000-fach, 10.000-fach oder 100.000-fach höherer Affinität gebunden als andere Liganden. Liganden sind bevorzugt Ionen, kleine Moleküle, Nukleinsäuren (DNA oder RNA in einzel- oder doppelsträngiger Form) oder andere Proteine beliebiger Länge. Kleine Moleküle können zu jeder bekannten Klasse von Molekülen gehören. Sie sind bevorzugt Lipide, Fettsäuren, Purine, Pyrimidine, Zucker, Alkaloide, Aminosäuren, biogene Amine, Isoprenoide oder Steroide. Besonders bevorzugt ist das kleine Molekül ein Zucker, ganz besonders bevorzugt Glucose. Besonders bevorzugt ist der Rezeptor ein Lektin, ganz besonders bevorzugt Concanavalin A.

Lektine sind Proteine, die in der Lage sind, Kohlehydratstrukturen spezifisch zu binden. Sie sind an vielfältigen molekularen und zellulären Erkennungsprozessen in Tieren, Pflanzen und Bakterien beteiligt und haben keine enzymatische Funktion. Viele Lektine sind postranslational durch Glykosylreste modifiziert.

Concanavalin A ist ein Lektin, das α-D-Glucose und ähnliche Zucker binden kann, ohne diese enzymatisch umzusetzen. Das Monomer besteht aus 237 Aminosäuren und enthält Mangan und Calcium. Bei neutralem pH bildet es ein Tetramer, das im sauren Bereich in zwei Dimere zerfällt. Es kommt in besonders hoher Konzentration in der Schwertbohne *Canavalia ensiformis* vor. Concanavalin A hat bevorzugt die durch SEQ ID NO: 3 definierte Aminosäuresequenz.

Als "modulatorische Proteine" werden in dieser Anmeldung solche Proteine bezeichnet, die mit anderen Proteinen interagieren und dadurch die Aktivität dieser Proteine verändern. Die Folge kann eine reduzierte Aktivität des Interaktionspartners sein (Inhibition) oder eine gesteigerte Aktivität (Aktivierung). Der Bindungspartner des Modulators ist bevorzugt ein Enzym. In diesem Fall beeinflusst die Bindung des Modulators die Affinität für das Substrat oder die Geschwindigkeit der enzymatischen Umsetzung des Substrats. Ebenfalls bevorzugt ist der Bindungspartner ein Rezeptor. In diesem Fall kann der Modulator agonistisch wirken, d.h. die durch Bindung an den Rezeptor diesen aktivieren oder der Modulator kann als Antagonist an den Rezeptor binden, ohne diesen dabei zu aktivieren. Weiterhin bevorzugt ist der Bindungspartner ein Transkriptionsfaktor. In diesem Fall beeinflusst die Bindung des Modulators die Fähigkeit des Transkriptionsfaktors, seinerseits die Genexpression zu verändern.

Ein "Transkriptionsfaktor" im Sinne dieser Anmeldung ist ein Protein, dessen Aktivität darin besteht, die Expression eines oder mehrerer Gene in der Zelle zu fördern oder zu inhibieren.

"Bindeproteine" sind Proteine, die in der Lage sind, andere Moleküle (Liganden) spezifisch zu binden. Spezifische Bindung bedeutet, dass der Ligand mit signifikant höherer Affinität gebunden wird als andere Substanzen. Vorzugsweise wird der Ligand mit wenigstens 10-fach, 100-fach, 1000-fach, 10.000-fach oder 100.000-fach höherer Affinität gebunden als andere Liganden.

Unter dem Begriff "Membrantransporter" werden solche Proteine verstanden, die die Passage anderer Moleküle durch die Zellmembran ermöglichen oder erleichtern. Diese Proteine sind in der Zellmembran lokalisiert.

Zytoskelettproteine sind Proteine, die die räumliche Struktur einer Zelle stabilisieren und in Kombination mit Motorproteinen zelluläre Bewegungsvorgänge vermitteln. Zytoskelettproteine sind bevorzugt Actin, Intermediärfilamente und Mikrotubuli. Mit Actinfilamenten und Mikrotubuli assoziierte Motorproteine sind bevorzugt Kinesin, Dynein und Myosin.

Der Begriff "Stabilisierung" bezeichnet die Aufrechterhaltung der Struktur und/oder der Funktion eines isolierten Proteins. Da die Struktur eines Proteins wesentliche Voraussetzung für dessen Funktion ist, führt eine Stabilisierung der Proteinstruktur vorzugsweise zu einer Aufrechterhaltung der Funktion des Proteins. Die zu stabilisierende Struktur des Proteins ist vorzugsweise jene Struktur, die es in seiner natürlichen Umgebung, d.h. im Gewebe oder in der Zelle hat. Die erfindungsgemäße Zubereitung stabilisiert vorzugsweise die Sekundär-, Tertiär- oder Quartärstruktur des isolierten Proteins. Unter Stabilisierung wird im Rahmen dieser Erfindung die Stabilisierung eines statistisch signifikanten Anteils der Moleküle des isolierten Proteins verstanden. So soll die Wiederfindungsrate an funktionalem isoliertem Protein nach Verabreichung der erfindungsgemäßen Zubereitung vorzugsweise zumindest 50 %, zumindest 75 %, zumindest 80 % zumindest 90 %, zumindest 95 % oder, besonders bevorzugt, zumindest 99 % betragen. Die Wiederfindungsrate kann daher bestimmt werden als Anteil funktionaler Moleküle des isolierten Proteins an der Gesamtmenge der Moleküle des isolierten Proteins nach Zugabe der erfindungsgemäßen Zubereitung und Lagerung. Bevorzugt werden zur Messung der Wiederfindungsrate die Aktivitäten der das isolierte Protein enthaltenden Zubereitung unmittelbar vor oder nach Zugabe der erfindungsgemäßen Zubereitung und zu einem weiteren Zeitpunkt verglichen. Außerdem erfolgen Vergleiche mit Zubereitungen des isolierten Proteins ohne Zugabe der erfindungsgemäßen Zubereitung zu gleichen Zeitpunkten.

Die vorliegende Erfindung ermöglicht vorteilhaft die verbesserte Stabilisierung isolierter Proteine, insbesondere Rezeptoren. Wie Beispiel 5 zeigt, wird Concanavalin A durch die nichtbindende Zelllysatfraktion aus den Samen von *Canavalia ensiformis* besser stabilisiert als durch bovines Serumalbumin, das im Stand der Technik als Stabilisator für Proteine bekannt ist. Ein weiterer überraschender Vorteil der erfindungsgemäßen, ein isoliertes Protein stabilisierenden Zelllysatfraktion ist die Möglichkeit, diese zu autoklavieren, ohne dass ihre Aktivität beeinträchtigt wird. Für Anwendungen der erfindungsgemäßen, ein isoliertes Protein stabilisierenden Zelllysatfraktion im medizinischen Bereich ist dieses ein erheblicher Vorteil, da so die Keimfreiheit der Zelllysatfraktion sichergestellt werden kann.

In einer bevorzugten Ausführungsform der vorliegenden Erfindung werden das isolierte Protein und die das isolierte Protein stabilisierende Zelllysatfraktion aus dem Samen der Schwertbohne (*Canavalis ensiformis*) gewonnen.

In einer besonders bevorzugten Ausführungsform der vorliegenden Erfindung enthält die das isolierte Protein stabilisierende Zelllysatfraktion Präcanavalin. Wenn die das zu isolierende Protein produzierende Zelle in ihrer natürlichen Form kein oder nicht ausreichend Präcanavalin enthält, ist es bevorzugt, in dieser Zelle Präcanavalin rekombinant zu exprimieren. Die Zugabe von Präcanavalin zu einem Zelllysat ist ebenfalls bevorzugt.

Der Begriff "Präcanavalin" bezeichnet ein im Samen der Schwertbohne (*Canavalia ensiformis*) enthaltenes Protein. Präcanavalin-Monomere zeigen in der Gelelektrophorese unter denaturierenden Bedingungen ein Molekulargewicht von 49.000 Dalton. Durch Trypsin wird Präcanavalin in zwei Peptide von 24.000 bzw 25.000 Dalton Molekulargewicht gespalten. Unter Bedingungen, die eine Kristallisation dieser Spaltprodukte erlauben, wird das größere der beiden Peptide ein weiteres Mal durch Trypsin gespalten (Campbell Smith et l., 1982, Biochemical Characterization of Canavalin, the Major Storage Protein of Jack Bean, Plant Physiology, 70: 1199-1209). Vorzugsweise hat Canavalin eine Aminosäuresequenz wie in SEQ ID NO: 2 definiert. In einer bevorzugten Ausführungsform der vorliegenden Erfindung weist das Präcanavalin eine Sequenzidentität von wenigstens 60 %, 70 %, 80 %, 90 %, 95 %, 97 %, 98 % oder 99 % zu der durch SEQ ID NO: 2 definierten Aminosäuresequenz auf. Vorzugsweise wird Präcanavalin durch ein Polynukleotid mit der durch SEQ ID NO: 1 definierten Sequenz kodiert. Ein Verfahren zur Bereitstellung von auf gereinigtem Präcanavalin aus der Schwertbohne ist in Beispiel 1 beschrieben.

In einer weiteren bevorzugten Ausführungsform der vorliegenden Erfindung wird die ein isoliertes Protein stabilisierende Zelllysatfraktion weiter aufgearbeitet.

Diese Aufarbeitung besteht bevorzugt in der Zugabe von Protease-Inhibitoren und/oder der Bindung freier Metallionen durch Chelatoren. Weiterhin ist die Sterilisierung der das Protein stabilisierenden Zelllysatfraktion bevorzugt: Ganz besonders bevorzugt besteht die Aufarbeitung in der Anreicherung einer Subpopulation von das isolierte Protein besonders wirksam stabilisierenden Bestandteilen in der Zelllysatfraktion. Ebenfalls ganz besonders bevorzugt ist die Abtrennung von störenden Bestandteilen aus der Zelllysatfraktion. Eine solche Auftrennung der das Protein stabilisierenden Zelllysatfraktion wird eingesetzt, um aus besagter Zelllysatfraktion solche Moleküle zu entfernen, die die technische Anwendung des Proteins behindern. Zur Isolierung einer Subpopulation von Molekülen aus der das Protein stabilisierenden Zelllysatfraktion können alle dem Fachmann bekannten Verfahren zur Auftrennung von Molekülgemischen verwendet werden. Bevorzugt werden die Trennungsverfahren eingesetzt, die oben für die Aufreinigung von Proteinen beschrieben wurden.

Im Rahmen der der vorliegenden Erfindung zugrunde liegenden Studie hat sich herausgestellt, dass Präcanavalin besonders gut dazu geeignet ist, isolierte Proteine zu stabilisieren. Deswegen besteht die weitere Aufarbeitung der ein isoliertes Protein stabilisierenden Zelllysatfraktion vorzugsweise in der Anreicherung von Präcanavalin.

Präcanavalin wird bevorzugt durch ein Verfahren angereichert, welches die folgenden Schritte umfasst:
1. Suspension von Schwertbohnenmehl in geeignetem Puffer;
2. Chromatografische Abtrennung des Concanavalin A mit einer Superdex 200- Säule. Das Präcanavalin befindet sich in der nichtbindenden Fraktion;
3. Dialyse der nichtbindenden Fraktion gegen Wasser;
4. Lyophilisierung der nichtbindenden Fraktion;
5. Versetzen der nichtbindenden Fraktion mit saurem Fällungspuffer, bevorzugt 50 mM Na-Acetat, pH 4,4 und anschließende Zentrifugation;
6. Waschen des Sediments mit Aqua dest. und anschließende Zentrifugation;
7. Das verbleibende Pellet wird in geeignetem Puffer, bevorzugt 1 % (Gewicht/Gewicht) NaCl und 0,1 % (Gewicht/Gewicht) K₂HPO₄, pH 7,0, aufgenommen und zentrifugiert;
8. Nach der Zentrifugation wird der Überstand gesammelt;
9. Das nach der Zentrifugation verbleibende Sediment wird in Kochsalzlösung, vorzugsweise 5 % (Gewicht/Gewicht) aufgenommen und erneut zentrifugiert;
10. Der in Schritt 9 erhaltene Überstand wird mit dem Überstand aus Schritt 8 vereinigt;
11. Dialyse der vereinigten Überstände gegen Aqua dest;
12. Aufkonzentration des Präcanavalins durch Säure-Base-Extraktion, bevorzugt durch Fällung mit 1 N Essigsäure, pH 5.1 und Aufnahme des Sediments in 0,01 N NaOH, wobei der pH nach der Aufnahme in der Base nicht mehr als 8,0 beträgt;
13. Dialyse gegen den für die anschließende Ionenaustauschchromatographie vorgesehenen Auftragspuffer, bevorzugt 20 mM Tris, 100 mM NaCL, pH 7,2;
14. Ionenaustauschchromatographie, bevorzugt mit einer Säule aus DEAE Sepharose FF und dem in Schritt 13 genannten Auftragspuffer, Elution bevorzugt mit steigendem Salzgradienten;
15. Identifikation der reinsten Fraktionen, bevorzugt mit SDS-PAGE, Vereinigung dieser Fraktionen;
16. Dialyse gegen Aqua dest.; und
17. Gefriertrocknung des Präcanavalin

Besonders bevorzugt wird Präcanavalin durch ein vereinfachtes Verfahren angereichert, welches die Schritte umfasst:
1. Suspension von Schwertbohnenmehl in geeignetem Puffer;
2. Ionenaustauschchromatographie, bevorzugt mit einer Säule aus DEAE-Sepharose FF, Elution bevorzugt mit steigendem Salzgradienten;
3. Identifikation der reinsten Fraktionen, bevorzugt mit SDS-PAGE, Vereinigung dieser Fraktionen;
4. Dialyse gegen Aqua dest.; und
5. Gefriertrocknung des Präcanavalins.

Eine weiter bevorzugte Ausführungsform der vorliegenden Erfindung betrifft die Verwendung einer Zubereitung enthaltend Präcanavalin zur Stabilisierung eines isolierten Proteins. Besonders bevorzugt ist die Verwendung der vorgenannten Zubereitung für die Stabilisierung von Concanavalin A.

Eine "Zubereitung enthaltend Präcanavalin" liegt vorzugsweise in flüssiger oder fester Form vor. Vorzugsweise enthält die Zubereitung außer Präcanavalin noch weitere Bestandteile aus der zellulären Umgebung des isolierten Proteins. Gleichfalls bevorzugt enthält die Zubereitung Chaperone, bovines Serumalbumin, und/oder kompatible Solute. Weiterhin bevorzugt enthält die Zubereitung organische oder anorganische Puffersubstanzen. Auch Konservierungsmittel, welche das Wachstum von Bakterien und Pilzen unterdrücken, und Benetzungsmittel sind als Bestandteile der erfindungsgemäßen Zubereitung bevorzugt. Der Gewichtsanteil von Präcanavalin an der Trockenmasse der erfindungsgemäßen Zubereitung beträgt vorzugsweise wenigstens 30 %, 40 %, 50 %, 60 %, 70 %, 80 %, 90 %, 95 %, 97 %, 98 %, 99 % oder 99,5 %.

In einer bevorzugten Ausführungsform, wird das in der erfindungsgemäßen Zubereitung enthaltene Präcanavalin rekombinant hergestellt.

Verfahren zur rekombinanten Herstellung von Proteinen sind dem Fachmann gut bekannt. Eine Nukleinsäuresequenz, die für Präcanavalin kodiert wird in einen Expressionsvektor eingebracht, der die Expression des Präcanavalins in dem gewählten Wirtsorganismus ermöglicht. Hiefür ist es bevorzugt, dass die Nukleinsäuresequenz, die für Präcanavalin kodiert unter Kontrolle einer geeigneten regulatorischen Sequenz steht. Geeignete regulatorische Sequenzen sind beispielsweise der lac-, trp- oder tac-Promotor für *E. coli*, der AOX1- oder GAL-1 Promotor für Hefen oder der CaMV-Promotor für Pflanzen. Für den Einsatz in tierischen Zellen sind der CMV-, SV40-, oder der RSV-promoter (Rous sarcoma virus) sowie der CMV-enhancer, der SV40-enhancer oder ein Globin-Intron bevorzugt. Bevorzugte Expressionsvektoren sind Plasmide, Phagen, retrovirale Vektoren und künstliche Chromosomen. Bevorzugte Wirtsorganismen für die rekombinante Herstellung von Präcanavalin sind Pflanzen, Prokaryoten und Pilze oder auch Säugetiere. Plasmide werden bevorzugt durch Elektroporation, Fällung mit Calciumphosphat oder Rubidiumchlorid oder durch Hitzeschock in die Wirtszelle eingebracht. Bevorzugte Verfahren für die Transformation von Pflanzenzellen sind die ballistische Inokulation und die durch *Agrobacterium tumefaciens* vermittelte Transformation.

Die vorliegende Erfindung betrifft weiterhin ein Verfahren zur Stabilisierung eines isolierten Proteins umfassend die Zugabe einer erfindungsgemäßen Präcanavalin enthaltenden Zubereitung zu dem isolierten Protein.

Die Zugabe der erfindungsgemäßen Präcanavalin enthaltenden Zubereitung zu dem gewünschten Protein ermöglicht die exakte Dosierung der erfindungsgemäßen Zubereitung. So kann eine definierte und reproduzierbare Stabilisierung des gewünschten Proteins erreicht werden. Bevorzugt wird die getrocknete Zubereitung als Feststoff in die das gewünschte Protein enthaltende Lösung gegeben und darin gelöst. Gleichfalls bevorzugt wird eine Stammlösung der erfindungsgemäßen Zubereitung der das gewünschte Protein enthaltende Lösung zugefügt. Besonders bevorzugt ist der Einbau der erfindungsgemäßen, Prävanavalin enthaltenden Zubereitung in Hydrogelpartikel, ganz besonders bevorzugt Alginatkugeln, wobei die Hydrogelparttikel auch das isolierte Protein enthalten.

In einer bevorzugten Ausführungsform des erfindungsgemäßen Verfahrens ist das isolierte Protein ein Rezeptor, stärker bevorzugt ein Lektin und noch stärker bevorzugt Concanavalin A.

Weiterhin betrifft die vorliegende Erfindung die Verwendung der erfindungsgemäßen ein isoliertes Protein stabilisierenden Zelllysatfraktion zur Stabilisierung eines isolierten Proteins, wobei besagtes isoliertes Protein Bestandteil eines biochemischen Sensors ist.

Ein "biochemischer Sensor" enthält ein Molekül, vorzugsweise ein Protein, das in der Lage ist, die nachzuweisende Substanz mit hoher Affinität zu binden. Dieses Protein wird im Folgenden als "Rezeptor" bezeichnet. Ein erfindungsgemäß hergestellter biochemischer Sensor enthält wenigstens einen Rezeptor sowie die Moleküle, die in der den Rezeptor/die Rezeptoren stabilisierenden Zelllysatfraktion enthalten sind. Der Fachmann weiß, dass ein biochemischer Sensor darüber hinaus weitere Moleküle enthalten kann.

Die Bindung des Analyten an den Rezeptor ermöglicht den qualitativen und bevorzugt auch den quantitativen Nachweis des Analyten in einer Probe. Unter qualitativem Nachweis wird die Feststellung der An- oder Abwesenheit des Analyten in der Probe verstanden. Der Fachmann weiß, dass der qualitative Nachweis abhängig vom verwendeten Sensor eine untere Nachweisgrenze hat, d.h. die Probe muss eine bestimmte Mindestkonzentration des Analyten aufweisen, damit dessen Anwesenheit festgestellt werden kann. Ein quantitativer Nachweis des Analyten liefert zusätzlich noch Informationen über die Menge oder Konzentration des Analyten in der Probe. Um die Wechselwirkung zwischen Rezeptor und Analyten messbar zu machen ist es bevorzugt, das verwendete Protein chemisch zu modifizieren. Besonders bevorzugt ist die Modifikation eines isolierten Proteins durch Markierung mit Fluoreszenzfarbstoffen.

Der Begriff "Probe" bezeichnet jede Flüssigkeit, in der der Analyt nachgewiesen werden soll. Bevorzugt sind Blut, Plasma, Serum, Tränenflüssigkeit, Gewebsflüssigkeit und Urin, besonders bevorzugt ist die Gewebsflüssigkeit unter der Bindehaut des Auges. Gleichfalls bevorzugt sind Gewebeproben. Die Probe liegt bevorzugt als aus dem Körper des Patienten isolierte Probe vor. Stärker bevorzugt liegt die Probe an ihrem natürlichen Ort vor. In diesem Fall wird der Sensor bevorzugt in den Körper des Patienten eingebracht und wird direkt an dem Ort eingesetzt, an dem sich die Probe befindet.

In einer bevorzugten Ausführungsform der Erfindung enthält der biochemische Sensor Concanavalin A als Rezeptor. Solche biochemischen Rezeptoren können zur Bestimmung der Glucose-Konzentration in Proben eingesetzt werden, da Concanavalin A Glucose bindet. In diesem Fall enthält der biochemische Sensor bevorzugt Hydrogele zusätzlich zum Rezeptor und der den Rezeptor stabilisierenden Zelllysatfraktion. Bevorzugt sind Hydrogele aus synthetischen Molekülen, Biomolekülen oder modifizierten Biomolekülen. Bevorzugte synthetische Moleküle sind Polyacrylate, Polyacrylamide und Polyvinylalkohole. Bevorzugte Biomoleküle sind Gelatine, Carageenan, Agarose, Amylose, Amylopectin, Alginate, Gellan, Cellulose und Cellulose-Derivate. Besonders bevorzugt sind Polyvinylalkohole und Alginat. Wenn der biochemische Sensor besagte Hydrogele enthält, liegen Rezeptor und Analyt in heterogener Phase vor.

In einer besonders bevorzugten Ausführungsform enthält der biochemische Sensor Hydrogelpartikel, in denen die das isolierte Protein stabilisierende Zelllysatfraktion, Dextran und Concanavalin A enthalten sind.

Weiterhin betrifft die vorliegende Erfindung die Verwendung einer erfindungsgemäßen ein isoliertes Protein stabilisierenden Zelllysatfraktion oder einer Präcanavalin enthaltenden Zubereitung zur Herstellung eines biochemischen Sensors.

Die Erfindung stellt auch einen biochemischer Sensor umfassend ein isoliertes Protein und eine das isolierte Protein stabilisierende Zelllysatfraktion, wie zuvor definiert, bereit.

Bevorzugt umfasst der biochemische Sensor ein Rezeptor als isoliertes Protein, besonders bevorzugt Concanavalin A. Bevorzugt ist die Zelllysatfraktion, zusammen mit Dextran und dem isolierten Protein, bevorzugt dem Rezeptor und besonders bevorzugt Concanavalin A, in Hydrogelpartikeln enthalten.

Ebenso wird durch die Erfindung ein biochemischer Sensor umfassend Concanavalin A und Präcanavalin bereitgestellt. Bevorzugt ist das Concanavalin A und das Präcanavalin zusammen mit Dextran in einem Hydrogelpartikel enthalten.

Die folgenden Ausführungsbeispiele dienen nur dazu, die Erfindung zu illustrieren. Sie sollen den Gegenstand der Patentansprüche in keiner Weise beschränken.

### Beispiele:

### Beispiel 1: Gewinnung der nichtbindenden Fraktion aus Schwertbohnen

300 g Schwertbohnenmehl (Canavalia ensiformis) werden in 100 mM Puffer suspendiert und zur Entfernung von Membranbestandteilen filtriert. Insgesamt werden 30 g Protein auf eine Superdex 200-Säule aufgetragen und die nicht an das Säulenmaterial bindende Proteinfraktion wird gesammelt. Insgesamt erhält man 3 Liter nichtbindende Fraktion (nbF). Die Lösung wird gegen Wasser dialysiert. Anschließend erfolgt eine Gefriertrocknung (Lyophilisation). Die lyophilisierte Substanz kann im Arbeitspuffer aufgenommen werden.

### Beispiel 2: Aufreinigung von Präcanavalin

### Extraktion einer Rohfraktion Präcanavalin aus der nichtbindenden Fraktion

8,0 g dialysiertes und gefriergetrocknetes nbF wird mit Fällungspuffer (50 mM Na-Acetat, pH 4,4) versetzt, inkubiert und anschließend zentrifugiert. Das Sediment der Fällung wird mit Wasser gewaschen und wiederum zentrifugiert. Das Pellet wird in 800 ml Salz-Hydrogenphosphatlösung (1% (w/v) NaCl + 0,1% (w/v) K₂HPO₄, pH 7,0) gelöst. Der Überstand nach Zentrifugation wird gesammelt. Das Sediment wird in 500 ml 5%iger Kochsalzlösung gelöst und anschließend zentrifugiert. Die vereinigten Überstände der Salzextraktionen werden gegen Aqua bidest dialysiert. Das Dialysat wird zentrifugiert . Das Sediment wird verworfen und der Überstand mit 1N Essigsäure auf pH 5,1 zur Präzipitation eingestellt. Der Präzipitationsansatz wird über Nacht inkubiert und am nächsten Tag zentrifugiert. Das Sediment wird in 50 ml 0,01N NaOH aufgenommen und mit 6 ml 0,1N NaOH versetzt, so dass eine nahezu klare Lösung vorliegt. Der pH-Wert wird überprüft uns sollte nicht mehr als pH 8,0 betragen. Der Ansatz wird bei 4°C über Nacht inkubiert. Der Säure-Base Extraktionsschritt wird einmal wiederholt. Das zuletzt erhaltene basische Präcanavalinextrakt wird gegen 20 mM Tris-Puffer, 100 mM NaCl, pH 7,2 dialysiert.

### Reinigung von Präcanavalin

Die Reinigung des Präcanavalins erfolgt über DEAE-Anionentauscherchromatographie (DEAE Sepharose FF). Das Protein wird auf die mit Auftragspuffer (20 mM Tris-Puffer, 100 mM NaCl, pH 7,2) äquilibierte Säule aufgetragen, mit Puffer nachgewaschen bis die Basis-Absorptionslinie bei 280 nm stabil ist und anschließend eluiert (Elutionspuffer: 20 mM Tris-Puffer, 1 M NaCl, pH 7,2). Die Elution erfolgt mit einem Salzgradienten von 0-100% . Nach der Analytik der Elutionsfraktionen mittels SDS-PAGE erfolgt die Vereinigung der reinsten Fraktionen. Das gereinigte Präcanavalin wird gegen Aqua bidest über Nacht dialysiert und dann gefriergetrocknet.

### Beispiel 3: Herstellung von Hydrogelpartikeln mit nichtbindender Fraktion (oder Präcanavalin)

2 g Alginsäure-Natriumsalz und 8 g nichtbindende Fraktion (oder Präcanavalin) werden in einem 250 ml Erlenmeyerkolben in 200 g Wasser unter Rühren gelöst. In einem 5L Becher werden 66,2 g CaC12 x 2H2O in 4931,3 g Wasser gelöst.

Die Alginat/Protein-Lösung wird über eine Pumpe in eine Zwei-Stoffdüse gefördert. Gleichzeitig wird am zweiten Eingang der Düse Druckluft angelegt, sodass die Alginat/Protein-Lösung in feine Tröpfchen zerstäubt wird. Die Tröpfchen werden durch die Luftströmung in ein Ultraschallbad mit der Calciumchlorid Lösung getragen, wo sie gelierten und zu Boden sinken. Die gelierten Kugeln werden anschließend gesammelt und ggf. autoklaviert.

### Beispiel 4: In vitro Stabilitätstest von Glucosesensoren

Glucosesensoren werden in jeweils 1 ml physiologischem Puffer bei 37°C gelagert. Nach entsprechender Lagerdauer wird der Glucosesensor entnommen und das Fluoreszenzspektrum bei verschiedenen Glucosekonzentrationen bestimmt. Die Veränderung der Fluoreszenzintensitäten mit steigendem Glucosegehalt dient als Maß für die Güte des Glucosesensors. Sensoren verschiedener Lagerdauern werden mit ungelagerten Sensoren verglichen und die absolute und prozentuale Abnahme der Reaktion auf Glucose über die Lagerdauer bestimmt.

Tabelle 1 zeigt vergleichend die Sensorantwort von mit nichtbindender Fraktion und mit Präcanavalin stabilisierten Sensoren im physiologischen Glucosebereich von 50 bis 250 mg/dL, ausgedrückt als prozentuale Signaländerung pro mg/dL Erhöhung der Glucosekonzentration. Nichtbindende Fraktion und Präcanavalin liefern identische Ergebnisse.

**Tabelle 1: Lagerfähigkeit stabilisierter Glucosesensoren**

| **Stabilisator** | **Sensorresponse im physiologischen Glucosebereich zwischen 50 und 250 mg/dL Glucose nach verschiedenen Lagerdauern [ Signaländerung / mg/dL Glucose]** | | |
|---|---|---|---|
| | 14 Tage | 60 Tage | 120 Tage |
| nichtbindende Fraktion | 0,46 | 0,29 | 0,22 |
| Präcanavalin | 0,44 | 0,29 | 0,20 |

### Beispiel 5: Ergebnis des Stabilitätstests mit verschiedenen möglichen Stabilisatoren relativ zu nicht stabilisiertem Sensor

Tabelle 2 zeigt Werte nach 120 Tagen Lagerung unter physiologischen Bedingungen. Während bekannte Stabilisatoren wie BSA, HSA das Sensorsystem nicht stabilisieren, wird durch nichtbindende Fraktion aus Bohnenmehlextrakt (auch autoklaviert) eine deutliche Stabilisierung erzielt.

**Tabelle 2: Vergleich verschiedener Stabilisatoren**

| **Stabilisator** | **Verbesserung Sensorresponse nach 120 Tagen Lagerung im Vergleich zu nicht stabilisiertem System** |
|---|---|
| HSA | 2 % |
| BSA | -3 % |
| nichtbindende Fraktion | 41 % |
| autoklavierte nichtbindende Fraktion | 27 % |

## Patentansprüche

1. Verwendung einer Zelllysatfraktion zur Aufrechterhaltung der Struktur und/oder der Funktion eines isolierten Proteins, wobei die Zelllysatfraktion durch ein Verfahren gewonnen wird, das die Schritte umfasst:
a) Gewinnung des Zelllysats aus Zellen; und
b) Abtrennung des isolierten Proteins aus dem Zelllysat, wodurch die Zelllysatfraktion zur Aufrechterhaltung der Struktur und/oder der Funktion des isolierten Proteins erhalten wird,
wobei das isolierte Protein Concanavalin A ist und die Zelllysatfraktion Präcanavalin enthält.

2. Verwendung gemäß Anspruch 1, wobei das isolierte Protein und die Zelllysatfraktion aus *Canavalia ensiformis* gewonnen werden.

3. Verwendung nach einem der Ansprüche 1 oder 2, wobei die Zelllysatfraktion nach der Abtrennung des isolierten Proteins weiter aufgearbeitet wird.

4. Verwendung nach einem der Ansprüche 1 bis 3, wobei die Zelllysatfraktion und das isolierte Protein Bestandteile eines biochemischen Sensors sind und das isolierte Protein die nachzuweisende Substanz mit hoher Affinität bindet.

5. Verwendung nach Anspruch 4, wobei der biochemische Sensor zusätzlich Dextran und Concanavalin in Hydrogelpartikeln umfasst.

6. Verwendung einer Präcanavalin enthaltenden Zubereitung zur Stabilisierung von Concanavalin A.

7. Biochemischer Sensor zur Bestimmung der Glukosekonzentration in einer Probe, umfassend ein isoliertes Protein, das die nachzuweisende Substanz mit hoher Affinität bindet, und eine Zelllysatfraktion, wobei die Zelllysatfraktion die Struktur und/oder die Funktion des isolierten Proteins aufrechterhält, wie in einem der Ansprüche 1 bis 3 definiert, wobei das isolierte Protein Concanavalin A ist und die Zelllysatfraktion Präcanavalin enthält, und wobei die Zelllysatfraktion zusammen mit Dextran und Concanavalin A in Hydrogelpartikeln enthalten ist.

8. Biochemischer Sensor nach Anspruch 7, wobei das Concanavalin A und das Präcanavalin zusammen mit Dextran in einem Hydrogelpartikel enthalten sind.

## Claims

1. Use of a cell lysate fraction for maintaining the structure and/or the function of an isolated protein, wherein the cell lysate fraction is produced by a method that comprises the steps:
a) producing the cell lysate from cells; and
b) separating off the isolated protein from the cell lysate, whereby the cell lysate fraction for maintaining the structure and/or the function of the isolated protein is obtained,
wherein the isolated protein is concanavalin A and the cell lysate fraction contains precanavalin.

2. Use according to Claim 1, wherein the isolated protein and the cell lysate fraction are produced from *Canavalia ensiformis*.

3. Use according to either of Claims 1 and 2, wherein the cell lysate fraction is further processed after the isolated protein is separated off.

4. Use according to any one of Claims 1 to 3, wherein the cell lysate fraction and the isolated protein are components of a biochemical sensor and the isolated protein binds the substance to be detected with high affinity.

5. Use according to Claim 4, wherein the biochemical sensor in addition comprises dextran and concanavalin in hydrogel particles.

6. Use of a precanavalin-containing preparation for stabilizing concanavalin A.

7. Biochemical sensor for determining the glucose concentration in a sample, comprising an isolated protein which binds the substance to be detected with high affinity, and a cell lysate fraction, wherein the cell lysate fraction maintains the structure and/or the function of the isolated protein, as defined in any one of Claims 1 to 3, wherein the isolated protein is concanavalin A and the cell lysate fraction contains precanavalin, and wherein the cell lysate fraction is contained together with dextran and concanavalin A in hydrogel particles.

8. Biochemical sensor according to Claim 7, wherein the concanavalin A and the precanavalin are contained together with dextran in a hydrogel particle.

## Revendications

1. Utilisation d'une fraction de lysat cellulaire pour le maintien de la structure et/ou de la fonction d'une protéine isolée, la fraction de lysat cellulaire étant obtenue par un procédé qui comprend les étapes suivantes :
a) l'obtention du lysat cellulaire à partir de cellules ; et
b) la séparation de la protéine isolée à partir du lysat cellulaire, la fraction de lysat cellulaire pour le maintien de la structure et/ou de la fonction de la protéine isolée étant obtenue,
la protéine isolée étant la concanavaline A et la fraction de lysat cellulaire contenant de précanavaline.

2. Utilisation selon la revendication 1, dans laquelle la protéine isolée et la fraction de lysat cellulaire sont obtenues à partir de *Canavalia ensiformis*.

3. Procédé selon l'une quelconque des revendications 1 ou 2, dans lequel la fraction de lysat cellulaire est davantage traitée après la séparation de la protéine isolée.

4. Utilisation selon l'une quelconque des revendications 1 à 3, dans laquelle la fraction de lysat cellulaire et la protéine isolée sont des constituants d'un capteur biochimique et la protéine isolée se lie à la substance à détecter avec une affinité élevée.

5. Utilisation selon la revendication 4, dans laquelle le capteur biochimique comprend en outre du dextrane et de la concanavaline dans des particules d'hydrogel.

6. Utilisation d'une préparation contenant de la précanavaline pour la stabilisation de concanavaline A.

7. Capteur biochimique pour la détermination de la concentration en glucose dans un échantillon, comprenant une protéine isolée, qui se lie à la substance à détecter avec une affinité élevée, et une fraction de lysat cellulaire, la fraction de lysat cellulaire maintenant la structure et/ou la fonction de la protéine isolée, telle que définie dans l'une quelconque des revendications 1 à 3, la protéine isolée étant la concanavaline A et la fraction de lysat cellulaire contenant de la précanavaline, et la fraction de lysat cellulaire étant contenue conjointement avec du dextrane et de la concanavaline A dans des particules d'hydrogel.

8. Capteur biochimique selon la revendication 7, dans lequel la concanavaline A et la précanavaline sont contenues conjointement avec du dextrane dans une particule d'hydrogel.
